# EUROPÄISCHE PATENTANMELDUNG

(11) **EP 4 403 544 A1**
(43) Veröffentlichungstag der Anmeldung: **24.07.2024**
(21) Anmeldenummer: 23151888.7
(22) Anmeldetag: 17.01.2023
(51) Int. Cl.: C07C 37/02, C07C 39/07

(54) **VERFAHREN ZUR HERSTELLUNG VON KRESOLEN**

(71) Anmelder: LANXESS Deutschland GmbH, 50569 Köln (DE)
(72) Erfinder: Kanthak, Matthias, 51379 Leverkusen (DE); Kaese, Thomas, 51375 Leverkusen (DE); Halle, Olaf, 51061 Köln (DE)

(57) **Zusammenfassung**

Die vorliegende Erfindung betrifft ein Verfahren zur Herstellung von Kresolen.

## Beschreibung

Die vorliegende Erfindung betrifft ein Verfahren zur Herstellung von Kresolen.

### Stand der Technik

Kresole werden häufig als Grundrohstoffe für die organische Synthese in den Bereichen Medizin und Chemie verwendet, z.B. zur Herstellung von Kunstharzen, Lokalanästhetika und Desinfektionsmitteln.

Großtechnisch werden Kresole vor allem durch die Reaktion von Chlortoluol mit Natronlauge unter hohen Temperaturen (~250 °C) und Druck (~300 bar) in einem Rohrreaktor synthetisiert, siehe Ullmann's Encyclopedia of Industrial Chemistry, 2012, Vol. 10, S. 427 - 428, Wiley-VCH Verlag GmbH & Co. KGaA, Weinheim. Bei der Umsetzung des Halogentoluols mit Halogen = Chlor und/oder Brom in einer wässrigen NaOH-Lösung entsteht zunächst Kresolat, welches dann mittels Zugabe einer Säure in Kresol umgewandelt wird. Im Zuge der Zugabe von Säure bilden sich 2 Phasen aus, eine kresolhaltige, in der Regel wasserunlösliche Phase und eine wässrige Phase.

Aus KR 10-1753291 ist ein Verfahren zur Herstellung von Kresolen bekannt, bei dem Kresole aus Kresolaten im stark sauren Bereich von maximalen pH-Werten von -5 bis 1 hergestellt werden. Dieses Verfahren hat den Nachteil, dass es große Mengen an Säure benötigt, um von den pH-Werten aus der Herstellung der Kresolate zu den pH-Werten ≤ 1 zu gelangen und, dass in diesem stark sauren Bereich viele Nebenprodukte, insbesondere Benzoesäure, in der kresolhaltigen Phase anfallen. Bei den so hergestellten Kresolen handelt es sich um ein Gemisch aus den Isomeren o-Kresol, m-Kresol und p-Kresol.

Die Trennung der einzelnen Isomere erfolgt im Anschluss an die Herstellung durch Destillation, wobei zunächst o-Kresol destillativ von m-/p-Kresol abgetrennt wird. Die Trennung von m-/p-Kresol kann aufgrund der fast identischen Siedepunkte in der Regel erst nach chemischer Derivatisierung erfolgen, idealerweise nach vorheriger Butylierung. Dabei erweisen sich die bei der Herstellung anfallenden Nebenprodukte, wie insbesondere Benzoesäure, als sehr nachteilig.

### Aufgabe der vorliegenden Erfindung

Aufgabe der vorliegenden Erfindung war daher die Bereitstellung eines verbesserten Verfahrens zur Herstellung von Kresolen, welches wirtschaftlich ist, eine hohe Ausbeute an Kresolen ermöglicht und bei dem wenig Benzoesäure anfällt.

### Lösung der Aufgabe

Überraschenderweise wurde nun gefunden, dass die der Erfindung zugrunde liegenden Aufgabe gelöst werden, wenn die Herstellung von Kresolen so erfolgt, dass nach der **Umsetzung** von **Halogentoluol** mit Halogen = Chlor und/oder Brom in einer **basischen wässrigen Lösung Säure** zugegeben wird, bis ein pH-Wert von 2 bis 7, vorzugsweise 3 bis 6,5, besonders bevorzugt 3,5 bis 6,2, eingestellt ist.

### Gegenstand der Erfindung

Gegenstand der Erfindung ist ein Verfahren zur Herstellung von **Kresolen** nach der **Umsetzung** von **Halogentoluol** mit Halogen = Chlor und/oder Brom mit einer **basischen wässrigen Lösung,** und anschließender Zugabe einer **Säure,** wobei die Zugabe einer Säure solange erfolgt bis ein pH-Wert von 2 bis 7, vorzugsweise 3 bis 6,5, besonders bevorzugt 3,5 bis 6,2, eingestellt ist.

Der Begriff **Kresole** im Sinne der Erfindung umfasst **o-Kresol, m-Kresol** und **p-Kresol** als Einzelverbindung sowie als Gemisch. Gemische können dabei in beliebigen Verhältnissen von o-, m-, und p-Kresol zueinander vorliegen.

**Halogentoluole** im Sinne der Erfindung umfassen Verbindungen, in der ein beliebiger Wasserstoff in einem Benzolring des Toluols durch ein Halogenatom mit Halogen = Chlor (Cl) oder Brom (Br) ersetzt wurde. Der Begriff Halogentoluole umfasst dabei die Ortho-, Meta- und Para-Halogentoluole einzeln, aber auch im Gemisch. Als Halogentoluole im Sinne der Erfindung können jegliche im Stand der Technik verfügbare Halogentoluole eingesetzt werden. Diese können aber auch beispielsweise hergestellt werden über die Umsetzung von Toluol mit Chlor.

**Basische wässrige Lösung** im Sinne der Erfindung umfasst die wässrige Lösung von starken Basen oder Mischungen starker Basen. Starke Base bedeutet dabei pK_{B} < 2. Bevorzugt sind Natriumhydroxid, Kaliumhydroxid, Calciumhydroxid und/oder Bariumhydroxid als starke Basen. Besonders bevorzugt sind NaOH und/oder KOH. Die basische wässrige Lösung hat dabei vorzugsweise eine Konzentration von 3-30 Gew.-%, besonders bevorzugt 4-20 Gew.-% an starker Base, im Wasser.

Die Umsetzung von **Halogentoluol** mit der **basischen wässrigen Lösung** wird vorzugsweise bei Temperaturen von 200 °C bis 500 °C und bei Drucken von 100 bar bis 500 bar durchgeführt, besonders bevorzugt bei 300 °C bis 480 °C und bei Drucken von 200 bar bis 500 bar.

**Säure** im Sinne der Erfindung umfasst starke Säuren oder Mischungen starker Säuren mit einem pKₐ-Wert von 2,5 oder weniger, vorzugsweise anorganische Säuren, besonders bevorzugt Salzsäure und/oder Schwefelsäure. Dabei sind Konzentrationen von 5 bis 40 Gew.-% starker Säuren in Wasser bevorzugt.

Die Zugabe von Säure erfolgt solange bis ein pH-Wert von 2 bis 7 erreicht ist. Bevorzugt sind pH-Werte von 3 - 6,5, besonders bevorzugt 3,5 - 6,2. Die Zugabe von Säure kann manuell oder automatisiert erfolgen, vorzugsweise unter statischer oder dynamischer Durchmischung bei 10-100 °C und einem Druck von 0,5-20 bar. Dabei sind alle Arten von Mischern und Mischaggregaten einsetzbar. Der pH-Wert kann dabei mit allen gängigen und handelsüblichen pH- Metern gemessen werden.

Der **pH-Wert** ist von der Messtemperatur abhängig. Die pH-Wert-Angabe im Rahmen dieser Erfindung bezieht sich auf eine Messung bei 25 °C.

Im Zuge der Einstellung des pH-Wertes von 2 bis 7, bevorzugt 3 - 6,5, besonders bevorzugt 3,5 - 6,2, bilden sich 2 Phasen aus, eine kresolhaltige Phase und eine wässrige Phase, die anschließend vorzugsweise voneinander getrennt werden.

### Detaillierte Beschreibung der Erfindung

Die einzelnen Schritte des erfindungsgemäßen Verfahrens lassen sich vorzugsweise wie folgt beschreiben:
Zunächst erfolgt die Umsetzung des Halogentoluols, vorzugsweise Chlortoluols, mit einer basischen wässrigen Lösung, vorzugsweise bei Temperaturen von 200 °C bis 500 °C und bei Drucken von 100 bar bis 500 bar, besonders bevorzugt bei 300 °C bis 480 °C und bei Drucken von 200 bar bis 500 bar.

Die basische wässrige Lösung enthält vorzugsweise 3-30 Gew.-% mindestens einer starken Base, besonders bevorzugt 4-20 Gew.-% mindestens einer starke Base, in Wasser. Vorzugsweise wird Natriumhydroxid, Kaliumhydroxid, Calciumhydroxid und/oder Bariumhydroxid eingesetzt, besonders bevorzugt sind Natriumhydroxid und/oder Kaliumhydroxid. Das molare Verhältnis von Natriumhydroxid und/oder Kaliumhydroxid zu Halogentoluol beträgt vorzugsweise 2-4 : 1, das molare Verhältnis von Calciumhydroxid und/oder Bariumhydroxid zu Halogentoluol beträgt vorzugsweise 1-2,5 : 1.

Durch die Reaktion eines Halogentoluols mit einer wässrigen basischen Lösung, wie beispielsweise einer 3-30 Gew.-%igen wässrigen NaOH, bilden sich Natrium- Kresolate.

Die Kresolate werden vorzugsweise in einem Rohrreaktor oder einem Druckautoklaven hergestellt und anschließend vorzugsweise abgekühlt auf Temperaturen von 100°C oder weniger und auf Atmosphärendruck entspannt, bevor die Umsetzung mit der Säure erfolgt.

Durch die Zugabe einer **Säure** wandeln sich die Kresolate in Kresole um und es erfolgt die Ausbildung von mindestens 2 Phasen, einer Kresol-haltigen Phase, die in der Regel wasserunlöslich ist, und einer wässrigen Phase, die auch noch Anteile an Kresolen enthalten kann, wobei vorzugsweise diese 2 Phasen voneinander **abgetrennt** werden.

Unter **Abtrennung** wird die Separation der Kresol-haltigen Phase von der wässrigen Phase verstanden. Die **Abtrennung** der Kresol-haltigen Phase von der wässrigen Phase kann dabei nach allen üblichen Verfahren erfolgen, wie beispielsweise mit Hilfe von Dekantern, Scheideflaschen oder Schütteltrichtern. Bevorzugt ist die Durchführung bei 1-10 bar, besonders bevorzugt unter Atmosphärendruck.

Die Kresol-haltige Phase kann gegebenenfalls weiter aufgereinigt werden, z.B. durch Verwendung eines ein oder mehrstufigen destillativen Verfahrens beispielsweise mittels Dünnschichtverdampfern, Fallfilmverdampfern, Umlaufverdampfern, Destillationsblasen, Rektifikationskolonnen, unter Vakuum oder Atmosphärendruck.

Die wässrige Phase kann gegebenenfalls noch Kresole enthalten. In einer weiteren Ausführungsform des erfindungsgemäßen Verfahrens erfolgt eine Zugabe eines organischen Lösungsmittels zu der abgetrennten wässrigen Phase, vorzugsweise um die verbleibenden Kresole aus der wässrigen Phase in eine organische Lösungsmittelschicht zu extrahieren. Im Anschluss an die Zugabe eines Lösungsmittels werden die lösungsmittelhaltige Schicht von der wässrigen Phase getrennt, vorzugsweise durch Separieren in einem Kessel, in einer Trennflasche oder Dekanter.

Als organisches Lösungsmittel wird vorzugsweise ein unpolares aromatisches Lösungsmittel eingesetzt, vorzugsweise Benzol, Toluol, Ethylbenzol, Xylol oder eine Mischung davon. Weiterhin sind Alkane oder Cylcoalkane sowie aliphatische Ether, Ketone und Ester bevorzugt wie beispielsweise Hexan, Heptan, Cyclohexan, Methyl-t-butylether (MTBE), Methylisobutylketon (MIBK), Isobutylacetat (iBA) oder eine Mischung davon.

In einer besonders bevorzugten Ausführungsform des erfindungsgemäßen Verfahrens wird Methyl-t-butylether (MTBE) oder Toluol als organisches Lösungsmittel eingesetzt.

In einer weiteren bevorzugten Ausführungsform des erfindungsgemäßen Verfahrens entspricht die Menge an zugesetztem organischen Lösungsmittel 1 Gewichtsteile bis 200 Gewichtsteile, besonders bevorzugt 5 Gewichtsteile bis 100 Gewichtsteile zugesetztes organisches Lösungsmittel, bezogen auf 100 Gewichtsteile der abgetrennten wässrigen Phase

Der Schritt der Zugabe eines organischen Lösungsmittels zu der wässrigen Phase zur Extraktion der Kresole wird vorzugsweise bei 0°C bis 100°C durchgeführt. Der **pH-Wert** der wässrigen Phase (bei 25°C) beträgt vorzugsweise 3 - 6,5, besonders bevorzugt 3,5 - 6,2.

Diese Zugabe eines organischen Lösungsmittels zu der wässrigen Phase zur Extraktion der Kresole kann einmal oder mehrmals durchgeführt werden.

Die Zugabe eines organischen Lösungsmittels zu der wässrigen Phase erfolgt vorzugsweise bei einem Druck von 1 bar bis 10 bar.

Diese Zugabe eines organischen Lösungsmittels zu der wässrigen Phase kann dabei als kontinuierliches Verfahren als auch als diskontinuierliches Verfahren durchgeführt werden. Als Verfahren kommen dabei alle Verfahren in Betracht, die ein Durchmischungs- und Trennungsvorgang beinhalten, beispielsweise das Mischen und anschließende Separieren in einem Kessel, das Separieren in einer Trennflasche oder Dekanter, die kontinuierliche Fahrweise in einer Mixer-Settler-Apparatur oder einer Füllkörperkolonne im Gegenstrom.

Es zeigt sich, dass sich Kresole mit dem erfindungsgemäßen Verfahren leichter und zuverlässiger bei pH-Werten von 2 -7 abtrennen lassen; die abgetrennten Kresole besitzen eine höhere Reinheit, durch weniger Nebenprodukte und vor allem durch einen geringeren Anteil an Benzoesäure.

Im Folgenden wird die Erfindung an Beispielen der vorliegenden Erfindung und Vergleichsbeispielen beschrieben. Die folgenden Beispiele sind jedoch nur ein bevorzugtes Beispiel für die vorliegende Erfindung und die vorliegende Erfindung ist nicht auf die folgenden Beispiele beschränkt.

### Ausführungsbeispiele:

### Umsetzung von Haloqentoluol mit wässrigem NaOH zur Herstellung Kresolaten:

Ein Kresolat-Gemisch (o-, m-, und p-Kresolat) wurde hergestellt, indem eine wässrige NaOH-Lösung (10 Gew.-%) und o-, m-, und p-Chlortoluol (Chlortoluol) in einen kontinuierlichen Reaktor mit einem molaren Verhältnis von 3 : 1 (NaOH : Chlortoluol) bei 400° C und 300 bar eingeführt wurde.

### Abtrennung der Kresole:

Zur Abtrennung der Kresole wurden mit Salzsäure in einer Konzentration von 20 Gew.-% die in der nachstehenden Tabelle angegebenen pH-Werte eingestellt. Die Temperatur betrug 25°C und der Druck 1 bar. Die kresolhaltige Phase wurde von der wässrigen Phase mittels Dekanter abgetrennt und die kresolhaltige Phase mittels Gaschromatographie analysiert. Die Ergebnisse sind in der nachstehenden Tabelle aufgeführt.

| **Bsp. Nr.** | **pH-Wert** | **Gehalt an Benzoesäure [Gew%]** | **Gehalt an Kresolen [Gew%]** | **Rest an Nebenprodukten [Gew%]** |
|---|---|---|---|---|
| **1 (V)** | 1 | 0,60 | 89,19 | 10,21 |
| **2 (erf.)** | 3,5 | 0,14 | 89,91 | 9,95 |
| **3 (erf.)** | 6 | 0,07 | 90,21 | 9,72 |

| | | | | |
|---|---|---|---|---|
| V = Vergleich, erf. = erfindungsgemäß, Rest = Leichtsieder (insbesondere Phenol) und Schwersieder (insbesondere Ditolylether, Tolylkresole) | | | | |

Es zeigte sich, dass die gezielte Einstellung des pH-Wertes von ≥ 2 zu einem deutlich verringerten Gehalt an Benzoesäure bei gleichzeitig hoher Ausbeute an Kresolen führt, im Vergleich zu der aus dem Stand der Technik genutzten Ansäuerung bei einem pH-Wert von 1.

## Patentansprüche

1. Verfahren zur Herstellung von **Kresolen** durch Umsetzung von **Halogentoluolen** mit Halogen = Chlor und/oder Brom mit einer **basischen wässrigen Lösung** und anschließender Zugabe einer **Säure,**
**dadurch gekennzeichnet, dass** die Zugabe einer Säure solange erfolgt bis ein **pH-Wert** von 2 bis 7, vorzugsweise 3 bis 6,5, besonders bevorzugt 3,5 bis 6,2 eingestellt ist.

2. Verfahren nach Anspruch 1, **dadurch gekennzeichnet, dass** es sich bei der Säure um Salzsäure und/oder Schwefelsäure handelt.

3. Verfahren nach Anspruch 1 oder 2, **dadurch gekennzeichnet, dass** es sich bei der **basischen wässrigen Lösung** um wässriges NaOH oder wässriges KOH handelt.

4. Verfahren nach einem der Ansprüche 1 bis 3, **dadurch gekennzeichnet, dass** die **basische wässrigen Lösung** eine Konzentration an Base von 3-30 Gew.% im Wasser aufweist.

5. Verfahren nach einem der Ansprüche 1 bis 4, **dadurch gekennzeichnet, dass** die Zugabe der Säure bei einer Temperatur von 0°C bis 100°C durchgeführt wird.

6. Verfahren nach einem der Ansprüche 1 bis 5, **dadurch gekennzeichnet, dass** sich nach der Zugabe der Säure eine kresolhaltige und eine wässrige Phase ausbilden, die voneinander getrennt werden und zu der abgetrennten wässrigen Phase mindestens ein organisches Lösungsmittel zugesetzt wird.

7. Verfahren nach Anspruch 6, **dadurch gekennzeichnet, dass** Methyl-t-butylether oder Toluol als organisches Lösungsmittel eingesetzt wird.
